# EUROPEAN PATENT SPECIFICATION

(11) **EP 3 491 942 B1**
(45) Date of publication and mention of the grant of the patent: **16.11.2022**
(21) Application number: 18205485.8
(22) Date of filing: 09.11.2018
(51) Int. Cl.: A24C 5/34, A24F 47/00

(54) **APPARATUS AND METHODS FOR COLLECTING EMISSIONS FROM A DEVICE**
VORRICHTUNG UND VERFAHREN ZUM SAMMELN VON EMISSIONEN AUS EINER VORRICHTUNG
APPAREIL ET PROCÉDÉS PERMETTANT DE COLLECTER DES ÉMISSIONS PROVENANT D'UN DISPOSITIF

(30) Priority: 16.11.2017 GB 201718953
(43) Date of publication of application: 05.06.2019
(73) Proprietor: Xyfil Limited, Preston, Lancashire PR1 1TP (GB)
(72) Inventor: Bhuva, Dilip, 400068 Mumbai (IN)
(74) Representative: Forresters IP LLP

(56) References cited:
- WO-A2-2013/060829
- US-A- 3 460 374
- US-A- 3 586 007
- US-A- 4 019 366

## Description

Embodiments of this invention relate to apparatus and methods for collecting emissions from a device. Particularly, although not exclusively, embodiments of the invention relate to apparatus and methods for drawing and collecting emissions from an Electronic Nicotine Delivery System (ENDS), such as an electronic cigarette, by using a pump. In some embodiments, the invention may also be suitable for drawing and collecting emissions from a smoking device, such as a traditional paper cigarette.

US 4019366 describes a variable parameter smoking machine for smoking cigarettes. The machine is operated with a pump providing the force for sucking air and smoke through a manifold. Gases passing into the manifold may be analysed by any suitable conventional means. Alternatively, gases may be analysed at any other point in the fluid circuit. WO 2013/060829 describes a system for nicotine analysis of a smoking article. During operation, air that is drawn through the smoking article is directed into a smoke analysis device which comprises an alkali flame ionisation detector for measuring the nicotine concentration. US 3460374 describes a machine for testing smoking cigarettes, in which a multi-ported solenoid valve controls smoke flow from the cigarette to the pump and from the pump to a smoke analyser unit. US 3586007 describes a cigarette smoking machine comprising a holder for one or more cigarettes, a low pressure condenser and a conductor connecting the holder to the condenser whereby large scale collection of the condensable components of the smoke from the cigarettes may be obtained for the purpose of analysis to determine the amount of such components inhaled by a cigarette smoker.

A reason for collecting emissions from an ENDS is so that the collected emissions can be tested to ensure that the emissions (and the liquids and/or device from which the emissions originate) comply with relevant jurisdictional regulations, such as the Tobacco Products Directive 2014/14/EU (TPD), Federal food, drug, and cosmetic Act" (FD&C Act), and The Family Smoking Prevention and Tobacco Control Act (Tobacco Control Act; TCA).

There are known types of apparatus for collecting emissions from traditional paper cigarettes so that the chemical constituents in the emissions can be determined by analysis. Such apparatus are typically operated so that emissions are drawn continuously from the traditional paper cigarette. One reason for drawing emissions continuously is that once traditional paper cigarettes have been lit (i.e. activated) they have a tendency to stop burning when not drawn from. This is particularly the case with fire-safe cigarettes, which are designed to slow or stop burning when not being used. Another reason for drawing emissions continuously is that this method can suitably provide a chemical constituent emissions profile which is indicative of the emissions that would otherwise have been exposed to a user. This is because the temperature at the tip of a burning paper cigarette remains relatively constant during its use. In other words, the temperature of the tip when emissions are being drawn is almost the same as the temperature of the tip when emissions are not being drawn. Therefore, the type and / or quantity of chemical constituents produced by a paper cigarette remain the same irrespective of whether or not the emissions from the paper cigarette are being drawn. It is, therefore, practical to draw emission continuously from a paper cigarette for the purposes of testing the profile of the chemical constituents therein.

Apparatus and methods for collecting ENDS emissions have not to the present day developed much beyond the techniques described above in relation to traditional paper cigarettes. However, it has been recognised that such techniques do not lend themselves well to collecting emissions from ENDS. One problem that has been identified is that ENDS are not designed to be drawn from continuously. In particular, the heating element (coil; which is used to heat / vapourise the liquid) is not designed to remain activate for long periods of time. Activating the coil for long periods of time can lead to premature expiration of the coil which can lead to contamination of the wick. Moreover, continuously drawing from an ENDS can result in the temperature of the heating element increasing up to a point where it can burn, melt, or short circuit. This can result in increased decomposition or cross-reaction of certain constituents of the e-liquid. For instance, it has been demonstrated that quantities of certain degradation products, such as aldehydes, ketones, and acrolein, can vary as a function of the temperature of the heating element. More problematic for testing, however, is that emissions that are produced when activating the coil for long periods of time are not representative of emissions that are produced (and inhaled) by a user when using an ENDS in real-life (i.e. when taking multiple short puffs typically separated by longer periods of inactivity). Since the purpose of collecting emissions from an ENDS is ultimately to determine the precise chemical profile of the emissions to which the end user is exposed to, it is imperative that the conditions that the liquids / emissions are exposed to during collection for testing is characteristic of the conditions that the liquids / emissions are exposed to during real-life use.

Most ENDS are also configured to shut-off after around 10 seconds of continuous activation. It is, therefore, impractical to utilise a conventional testing apparatus that is configured to draw continuously.

It is an object of the invention, therefore, to provide emissions collecting apparatus and methods that closely represent the puffing profile of real-life use.

A further problem that has been recognised is that constituents of ENDS liquids are somewhat of a relative unknown and differ from one product to another (i.e. from one flavour to another). There are many thousands of different ENDS liquids currently on the market and very little data showing what emissions are produced once these liquids have been atomised. Given the relative unknowns, it is important that the apparatus is capable of collecting as many of the chemical constituents (at whatever concentrations) in the emissions as possible so that a chemical constituent profile that is representative of real-life use can be determined.

It is a further object of the present invention, therefore, to provide emissions collecting apparatus and methods that improve the sensitivity of subsequent analysis step(s).

According to a first aspect of the invention we provide a collecting assembly comprising:
a condensing device including an inlet, an outlet and a spiral-shaped conduit extending between the inlet and the outlet;
characterised in that the collecting assembly further comprises:
a receptacle for collecting emissions from the condensing device, the receptacle having a side wall and a base, wherein the inner surface of the base defines a cone shape.

The spiral-shaped conduit may comprise a capillary.

The spiral-shaped conduit may have a narrower internal diameter at the outlet than at the inlet. In some cases, the internal diameter may reduce gradually along the length of the spiral-shaped conduit from the inlet to the outlet, e.g. in a tapered fashion.

Alternatively, the spiral-shaped conduit may have a constant internal diameter between the outlet and the inlet.

The internal diameter of the spiral-shaped conduit may be from approximately 2 mm to approximately 10 mm.

The collecting assembly may comprise a device configured to regulate the temperature of the receptacle.

Embodiments of the invention will now be described by way of example only and with reference to the accompanying drawings, of which:
Figure 1 is a schematic of an apparatus according to an embodiment that is not part of the invention as claimed;
Figure 2 is a schematic of an apparatus according to an embodiment that is not part of the invention as claimed;
Figure 3 is a schematic of an apparatus according to an embodiment that is not part of the invention as claimed;
Figure 4 is a schematic showing a controller and a pump assembly of an embodiment that is not part of the invention as claimed;
Figure 5 is a graph of data obtained by performing an emissions drawing cycle according to an embodiment that is not part of the invention as claimed;
Figure 6 is a graph of data obtained by performing an emissions drawing cycle according to an embodiment that is not part of the invention as claimed;
Figure 7 is a schematic of an apparatus according to an embodiment that is not part of the invention as claimed;
Figure 8 is a schematic of a part of an apparatus according to an embodiment that is not part of the invention as claimed;
Figure 9 is a schematic of a collecting assembly according to an embodiment; and
Figure 10 is a schematic of an apparatus according to an embodiment that is not part of the invention as claimed.

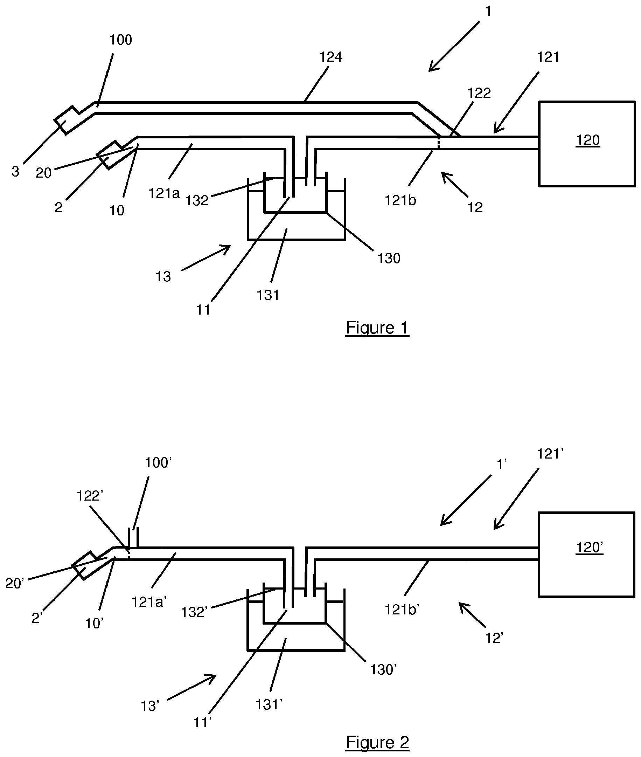

Referring to figure 1 there is shown an apparatus, indicated generally at 1, for collecting emissions from a device 2. The device 2 may be an ENDS (sometimes referred to as an EC, e-cigarette, e-cig, personal vaporiser or PV), an electronic non-nicotine delivery system (ENNDS), a traditional paper cigarette or a cigar.

The apparatus 1 comprises an inlet 10 for receiving emissions from the device 2. In some embodiments, the inlet 10 may be configured to mate (e.g. receive or be received by) an outlet 20 of the device 2.

In some embodiments, the inlet 10 and the outlet 20 may be connected by a conduit or other suitable connecting component (not shown). In such embodiments, the conduit / connecting component may function as an extension of the inlet - in other words, the free end of the conduit / connecting component to which the outlet 20 is connected may be the inlet 10 of the apparatus 1.

The apparatus 1 comprises an outlet 11 and a pump assembly, indicated generally at 12. The pump assembly 12 includes a pump 120 in fluid communication with the inlet 10. In some embodiments, the inlet 10 and the pump 120 are in fluid communication with one another via a fluid line, indicated generally at 121. The fluid line 121 may comprise one or more conduit(s), e.g. tube(s), 121a, 121b. The inlet 10 may be connected to the outlet 11 by conduit 121a such that emissions received by the inlet 10 can be channelled to the outlet 11 via the conduit 121a. Thus, as it will be appreciated, a partial vacuum created by the pump 120 within the fluid line 121 has the effect of drawing emissions from the device 2 to the outlet 11.

Advantageously, in some embodiments, the pump assembly 12 is configured such that, in use, emissions are drawn from the inlet 10 intermittently. There are numerous different ways in which the pump assembly 12 may be configured to draw emissions intermittently.

For instance, in some embodiments, the pump assembly 12 may include at least one valve 122. Including at least one valve 122 may be advantageous in embodiments where the pump 120 is a vacuum or suction pump configured to create a continuous partial vacuum.

In some embodiments, the or each valve 122 may be an electromechanical valve, such as a solenoid valve, or a manual valve.

In some embodiments, the pump assembly 12 may include one or more additional fluid line(s) 124 connecting the or each valve 122 to a further device 3 or a further inlet 100.

In some embodiments, the pump assembly 12 may be configured to draw emissions intermittently from the inlet 10 to the outlet 11 without the use of one or more valves 122. For instance, the pump 120 may be any one of a piston, plunger or peristaltic pump. In such embodiments, the pump 120 itself may be configured to draw emissions intermittently from the inlet 10 to the outlet 11.

In some embodiments, the apparatus 1 may include a collecting assembly, indicated generally at 13. The collecting assembly 13 may include a receptacle 130 for collecting emissions from the outlet 11. The collecting assembly 13 may include a device 131 configured to regulate the temperature of the receptacle 130. For instance, the device 131 may be configured to cool the receptacle 130. The device 131 may comprise a cooling bath of the type including a cooling agent (such as dry ice, ice or liquid nitrogen). In some embodiments, the device 131 may be configured to include an organic solvent or water. In some embodiments, the device 131 may comprise a thermoelectric cooler. It is to be appreciated that the device 131 is configurable to regulate the temperature of the receptacle 130, such that emissions drawn from the outlet 11 are condensed therein. Condensing emissions directly in the receptacle 130, rather than dissolving the emissions in one or more solvent(s) or collecting emissions via one or more filter pad(s) or by one or more thermal desorption unit(s), is advantageous in that the emissions (and thus the chemical constituents thereof) remain concentrated. Clearly, having concentrated emissions improves the sensitivity of subsequent analysis steps for determining the chemical composition of the emissions.

As illustrated by figure 1, in some embodiments a valve 122 may located between the outlet 11 and the pump 120.

An example of how the apparatus 1 can be operated will now be described.

As will be appreciated, as illustrated in figure 1, when in a first valve configuration (depicted by a solid line), the valve 122 permits drawing of emissions by the pump 120 from the inlet 10 to the outlet 11 and, when in a second valve configuration (depicted by a dashed line), the valve 122 prohibits or substantially prohibits drawing of the emissions by the pump 120 from the inlet 10 to the outlet 11. When in the second valve configuration (dashed line), fluid is drawn by the pump 120 from the further device 3 and along the fluid line 124.

In the illustrated embodiment, the valve 122 includes a single valve member that can be moved to block / open fluid lines 121, 124. In some embodiments, however, the valve 122 may include one or more additional valve members for controlling flow.

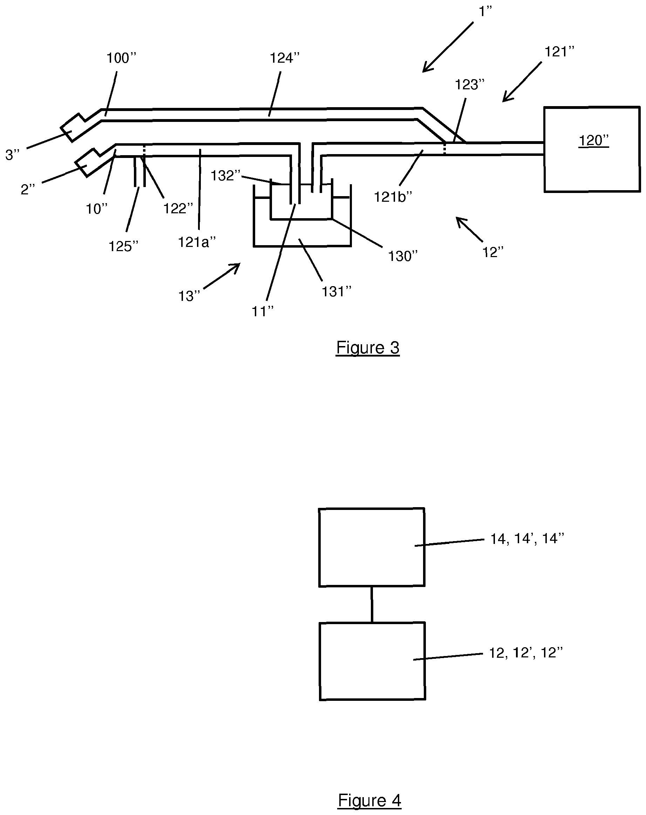

The device 2 may be connected to the outlet 11 whilst the valve 122 is in the second valve configuration (dashed line). The device 2 and the pump 120 may be switched on. As will be appreciated, when in the second valve configuration (dashed line), the valve 122 prohibits or substantially prohibits drawing of emissions from the device 2. Instead, fluid (i.e. gas) may be drawn from device 3 (which is typically switched off) along additional fluid line 124, which may act as a dummy line. In some embodiments, the device 3 need not be connected to the additional fluid line 124 and, instead, gas from atmosphere or a source of compressed gas may be drawn along the additional fluid line 124. Once the pump 120 is up to speed and the device 2 is ready for use the valve 122 may be switched to the first valve configuration (solid line), as shown in figure 1, for example by a controller 14 (see figure 4). As will be appreciated, switching the valve 122 to the first valve configuration (solid line) creates a partial vacuum within the conduits 121a, 121b, the receptacle 130 and at the inlet 10, which can cause activation of the device 2 and subsequent drawing of emissions from the inlet 10 to the outlet 11. A sealing member 132 (such as a rubber plug or sealing membrane) may be provided at the receptacle 130 and around the conduits 121a, 121b to maintain the partial vacuum, to avoid escape of emissions from within the receptacle 130 and / or conduits 121a, 121b, and also to avoid contamination from entering the emissions stream.

In some embodiments, the pump assembly 12 may be configured to perform an emissions drawing cycle over a predetermined time. The emissions drawing cycle may comprise multiple intervals of time in which emissions are drawn by the pump 120 from the inlet 10 to the outlet 11, for example when the valve 122 is in the first valve configuration (solid line). During periods of time between each interval, for example when the valve 122 is in the second valve configuration (dashed line), no emissions or a nominal volume of emissions are drawn by the pump 120 from the inlet 10 to the outlet 11. Clearly, alternating the configuration of the valve 122 between the first and second valve configurations in this manner has the effect that emissions are drawn from the device 2 to the outlet 11 intermittently (i.e. non-continuously).

Figure 2 shows an embodiment similar to that shown in figure 1, with the exception that the valve 122' is located between the inlet 10' and the outlet 11' (in the embodiment of figure 1 the valve 122 is located between the outlet 11 and the pump 120). In its first valve configuration (depicted by a solid line) the valve 122' permits drawing of emissions by the pump 120' from the inlet 10' to the outlet 11'. In its second configuration (depicted by a dashed line) the valve 122' prohibits or substantially prohibits drawing of emissions from the inlet 10' to the outlet 11'. Instead, whilst the valve 122' is in its second valve configuration (dashed line) fluid is drawn to the outlet 11' via the further inlet 100'.

In principal, the apparatus 1' of figure 2 operates in the same manner as described above in relation to figure 1. However, whilst the apparatus 1' shown in figure 2 operates adequately, it has been found that the apparatus 1 of figure 1 is an improvement (when compared to the apparatus 1' of figure 2) since it more closely represents the puffing profile of real-life use. The control of back pressure within the apparatus 1, 1' is of particular importance since the process is pressure sensitive. Increasing back pressure in the system can lead to an increase in back pressure in the device 2, 2' itself, which can have negative consequences. For instance, where the device 2, 2' is an ENDS, an increase in back pressure can lead to uncharacteristic wicking of emissions therefrom. Advantageously, by locating the valve 122 between the outlet 11 and the pump 120 (as shown in figure 1), build-up of back pressure within the apparatus 1 (and hence the device 2) is minimised. One key advantage of the apparatus 1 over the apparatus 1' is that substantially the entire fluid line 121 is isolated from the pump 120 when the valve 122 is in the second configuration (dashed line), thereby preventing contamination of the fluid line 121 between the device 2 and the valve 122. Isolating the fluid line 121 can also have the effect of minimising vaporisation of condensed sample from the receptacle 130. It can be appreciated that when valve 122' of apparatus 1' is in the second configuration (dashed line), there will be partial pressure between the valve 122' and the pump 120' (particularly in the receptacle 130') which has the potential to cause vaporisation of the collected emissions. Furthermore, airflow through the receptacle 130' will continue even when no emissions are being drawn from the device 2', thereby increasing chances of sample contamination as some of this passing air may condense in the receptacle 131'.

Figure 3 shows an embodiment similar to that shown in figures 1 and 2, with the exception that a first valve 122" is located between the inlet 10" and the outlet 11" and a second valve 123" is located between the outlet 11" and the pump 120". In their first valve configurations (depicted by a solid line) first valve 122" and the second valve 123" permit drawing of emissions by the pump 120" from the inlet 10" to the outlet 11". Switching either the first valve 122" or the second valve 123" to their respective second valve configurations (shown by a dashed line) has the effect of prohibiting or substantially prohibiting drawing of emissions by the pump 120" from the inlet 10" to the outlet 11".

An example of how the apparatus 1" can be operated will now be described.

The device 2" may be connected to the outlet 11" whilst the first valve 122" is in the second valve configuration (dashed line). The second valve 123" may also be in its respective second valve configuration (dashed line). A further device 3" may be connected to the inlet 100". The device 2" and the pump 120" may be switched on. As will be appreciated, when in the second valve configuration (dashed line), the second valve 123" prohibits or substantially prohibits the creation of a partial vacuum in the fluid line 121". Instead, fluid (i.e. gas) is drawn from device 3" (which is typically switched off) along additional fluid line 124", which may act as a dummy line. Once the pump 120" is up to speed and the device 2" is ready for use the second valve 123" may be switched to the first valve configuration (solid line), for example by the controller 14". As will be appreciated, switching the second valve 123" to the first valve configuration (solid line) creates a partial vacuum within the conduits 121a", 121b", the receptacle 130" and at additional fluid line 125". At this stage a partial vacuum is not created at the inlet 10" due to the fact that the first valve 122" is in its second valve configuration (dashed line), so no emissions are drawn from the device 2".

Whilst the first valve 122" remains in its second valve configuration (dashed line) the pump assembly 12" may be configured to start switching the second valve 123" between its first and second valve configurations. This is known as a calibration step in which the pressures and / or temperatures within the apparatus 1" are allowed to equilibrate. In this calibration step, a flowmeter may be connected to the additional fluid line 125" which can allow calibration of the pump 120" such that a desired flowrate is achieved (in the apparatus 1, 1' of previous figures 1 and 2 a desired flowrate can be achieved by attaching a flowmeter either at inlet 10, 10' or at inlet 100, 100', respectively). Once the calibration step has been completed the first valve 122" may be switched (either manually or by the controller 14") to its first valve configuration (solid line) so as to start performing an emissions drawing cycle by permitting drawing of emissions by the pump 120" from the inlet 10" to the outlet 11 ". The emissions drawing cycle may comprise multiple intervals of time in which emissions are drawn by the pump 120" from the inlet 10" to the outlet 11 ", for example when the valve 123" is in its first valve configuration (solid line). During periods of time between each interval, for example when the valve 123" is in its second valve configuration (dashed line), no emissions or a nominal volume of emissions are drawn by the pump 120" from the inlet 10" to the outlet 11 ".

Referring now to figure 4, there is shown the controller 14, 14', 14". In some embodiments, the controller 14, 14', 14" may be configured to output a control signal to operate the pump assembly 12, 12', 12". For instance, the control signal may operate the pump assembly 12, 12', 12" intermittently. Additionally or alternatively, the controller 14, 14', 14" may be configured to activate and / or deactivate the pump 120, 120', 120", control the speed of the pump 120, 120', 120", control the valve(s) 122, 122', 122", 123, 123', 123" or any combination thereof. In some embodiments, the controller 14, 14', 14" may be configured to control the device 131, 131', 131" so as to regulate the temperature of the receptacle 130, 130', 130". In some embodiments, the controller 14, 14', 14" may be configured to activate and / or deactivate the device 2, 2', 2", measure and / or record data on various aspects of the device (2, 2', 2") such as voltage, wattage, temperature, resistance and so on. In some embodiments, the controller 14, 14', 14" may be programmable. For instance, the controller 14, 14', 14" may be able to record data, store data, analyse data, transmit data, export data, report data, present data or any combination thereof. These data may be recordable, storable, analysable, transmittable, exportable, reportable or presentable in any format known to the skilled person, such as by graphs, or known file formats. The controller 14, 14', 14" may also be configurable to compare data between various devices, products (e.g. e-liquids), or experiments.

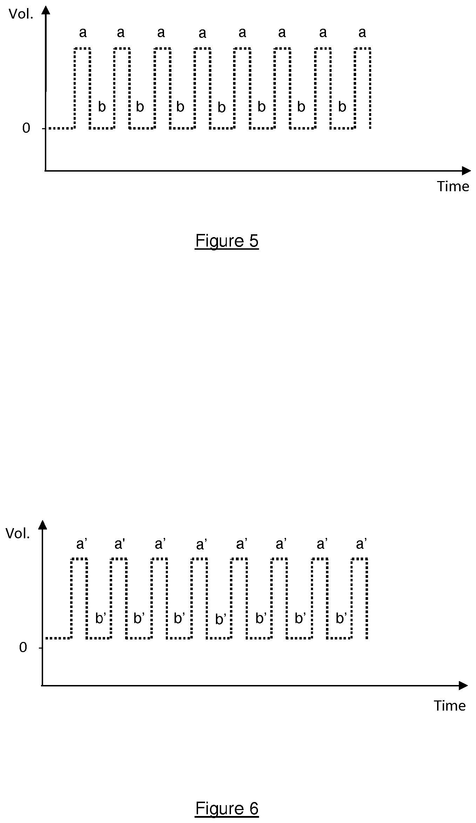

Figure 5 is a graph exemplifying an emissions drawing cycle according to an embodiment. The y-axis represents the volume of emissions drawn from a device 2, 2', 2" as a function of time (x-axis). The graph illustrates that the emissions drawing cycle includes multiple intervals of time (a) in which emissions are drawn by the pump 120, 120', 120" and periods of time (b) between each interval of time (a) in which no emissions are drawn by the pump 120, 120', 120".

Figure 6 is a graph exemplifying an emissions drawing cycle according to an embodiment. Again, the y-axis represents the volume of emissions drawn from a device 2, 2', 2" as a function of time (x-axis). The graph illustrates that the emissions drawing cycle includes multiple intervals of time (a') in which emissions are drawn by the pump 120, 120', 120" and periods of time (b') between each interval of time (a') in which a nominal volume of emissions are drawn by the pump 120, 120', 120". It is to be appreciated that the apparatus 1, 1', 1" may exhibit various degrees of tolerance and it may not be possible to entirely prohibit drawing of emissions from the device 2, 2', 2". Thus, the phrase "nominal volume" is intended to mean close to zero and within accepted tolerances of the apparatus 1, 1', 1".

It has been found that by drawing emissions from the device 2, 2', 2" intermittently, for example by performing an emissions drawing cycle according to embodiments, the collected emissions closely represent the emissions that would have been produced (and inhaled) by a user when using the device 2, 2', 2" in real life. In other words, drawing the emissions intermittently closely represents the puffing profile of real-life use, which can be beneficial in terms of getting an accurate understanding of the chemical profile of the collected emissions.

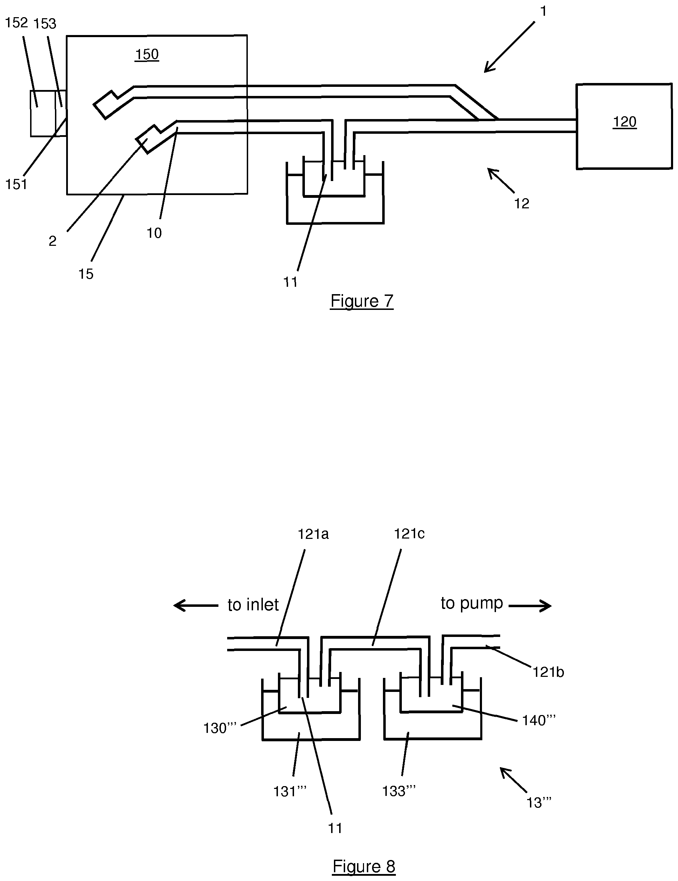

With reference to figure 7, in some embodiments, the apparatus 1 may comprise a chamber 15 defining an interior 150 that, in use, is substantially free or free of impurities. Whilst figure 7 shows the apparatus 1 of figure 1 having such a chamber 15, it is to be appreciated that any other embodiment, for instance apparatus 1' of figure 2 or apparatus 1" of figure 3, may also be provided with such a feature.

The inlet 10 (and preferably the device 2) is located within the interior 150 of the chamber 15. Since the interior 150 is substantially free of impurities, these configurations have the advantageous effect that emissions drawn to the outlet 11 contain no (or a negligible quantity of) impurities (generally derived from the atmosphere). Clearly, the sensitivity during subsequent analysis of the collected emissions is improved by reducing contamination in this way. This is especially relevant in countries where the air contains significantly high levels of pollution. Moreover, in certain countries the air contains significantly high levels of contaminants that are likely to be found in ENDS emissions. Therefore, it is important to remove the contaminants from the atmosphere in order to accurately determine what is in the ENDS emissions.

In some embodiments, the chamber 15 may define an opening 151 for receiving air from one or both of the atmosphere and a source of compressed air 152. The opening 151 may be occluded by a filter assembly 153, for example a canister of compressed air. The source of compressed air 152 may include compressed purified air.

In some embodiments, the filter assembly 153 may include any one or a combination of a particulate filter, an activated carbon filter, a compressed intake filter, other type(s) of adsorbent known to the skilled person, one or more absorbent or chemically active / reactive filter(s) (e.g. chemical traps, catalytic converter), and a drying agent. Consequently, air which is drawn into the interior 150 of the chamber 15 from the atmosphere and / or the source of compressed air 152 during operation of the apparatus 1 is free from contaminants, impurities, etc.

As shown in some embodiments, a collecting assembly 13'" may include a first receptacle 130‴ for collecting emissions from the outlet 11 and a second receptacle 140‴. The second receptacle 140‴ may be arranged in series with respect to the first receptacle 130‴, such that the second receptacle 140‴ can collect emissions that have not been collected by the first receptacle 130‴.

The collecting assembly 13‴ may be incorporated into any apparatus 1, 1', 1" that has been discussed herein.

In some embodiments, the collecting assembly 13‴ may include a device 131‴ configured to regulate the temperature of the first receptacle 130‴. The device 131‴ may be the same as discussed above in relation to other embodiments. In some embodiments (not shown), the device 131‴ may be configured to regulate the temperature of the second receptacle 140‴ (in addition to the first receptacle 130‴). In such embodiments, the first and second receptacles 130‴, 140‴ may be regulated at the same or at different temperatures to one another. For instance, the first receptacle 130‴ may be regulated at a temperature in the range of 0 °C to 4 °C and the second receptacle may be regulated at a temperature in the range of -10 °C to -20 °C or -82 °C to -76 °C.

In some embodiments, the collecting assembly 13‴ may include a second device 133‴ configured to regulate the temperature of the second receptacle 140‴. The first and second receptacles 130‴, 140‴ may be in fluid communication with one another via a conduit 121c.

As discussed, a reason for arranging first and second receptacles 130‴, 140‴ in series is such that they can be regulated at different temperatures to one another. Advantageously, this means that any emissions that are not condensed in the first receptacle 130‴ may still be condensed in the second receptacle 140‴, thereby maximising the yield that is collected. It has also been determined that yields can be improved when regulating such first and second receptacles 130‴, 140‴ at the same temperature to one another, when compared to systems having only a single receptacle. Clearly, collecting a high yield is beneficial insofar as it enables the determination of an accurate chemical profile of the emissions. Moreover, embodiments where the second receptacle 140‴ has a lower temperature than the first receptacle 130‴ can allow collection of chemicals that have very low boiling point. Also, the temperature difference between the two receptacles 130‴, 140‴ (i.e. stepdown cooling) can ensure that no blockage is created in the fluid line if some chemicals or components of the emissions are condensed and solidified, especially in the first receptacle 130"'. For example, (where there is no second receptacle 140‴) at temperature below 0 °C in the first receptacle 130‴, the inventor has observed that water vapour can condense and solidify (i.e. form ice crystals), thereby blocking the entire fluid line. The probability of blockages forming can be reduced by including the second receptacle 140‴ regulated at a temperature different from the first receptacle 130‴.

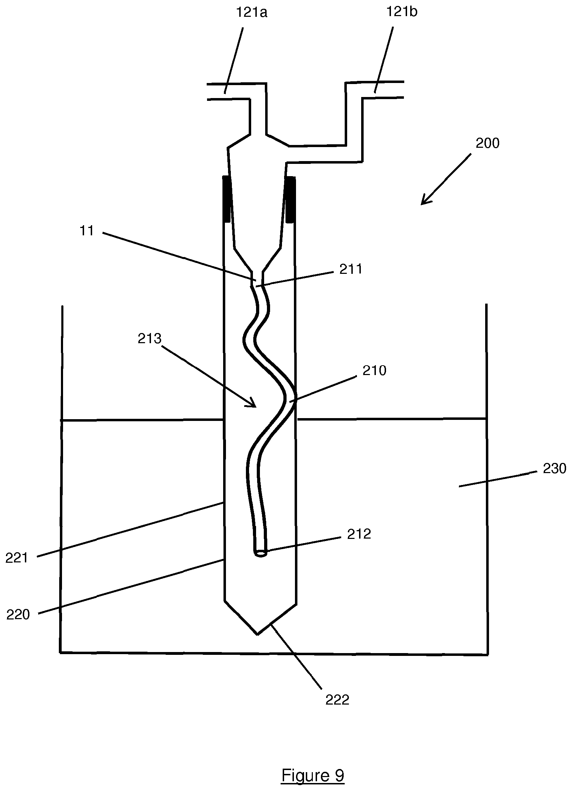

Figure 9 shows a collecting assembly, indicated generally at 200, according to an embodiment of the invention. The collecting assembly 200 comprises a condensing device 210 and a receptacle 220. In some embodiments, a device 230 configured to regulate the temperature of the receptacle 220 may be provided.

The condensing device 210 includes an inlet 211 in communication with the outlet 11 of the apparatus 1, so that emissions drawn from the device 2 can be transferred to the condensing device 210 and thence to the receptacle 220. The condensing device 210 includes an outlet 212 and a spiral-shaped conduit, indicated generally at 213, extending therebetween. Advantageously, the provision of a spiral-shaped conduit 213 allows more emissions to be condensed in the condensing device 210 when compared to conventional straight conduits, thereby improving the yield of condensed emissions collected by the receptacle 220.

In some embodiment, the spiral-shaped conduit 213 may comprise a capillary. In some embodiments, the spiral-shaped conduit 213 may have a narrower internal diameter at the outlet 212 than at the inlet 211. For instance, the internal diameter may reduce gradually, e.g. in a tapered fashion, along the length of the spiral-shaped conduit 213 from the inlet 211 to the outlet 212. An advantage of this arrangement is that molecules in the emissions passing between the inlet 211 and the outlet 212 are forced closer to one another, thereby increasing the efficiency of condensation.

In some embodiments, the spiral-shaped conduit 213 may have a constant internal diameter between the outlet 212 and the inlet 211.

The internal diameter of the spiral-shaped conduit may be from approximately 2 mm to approximately 10 mm. For instance, in embodiments where the spiral-shaped conduit 213 has a narrower internal diameter at the outlet 212 than at the inlet 211, the internal diameter approximate to the outlet 212 may be around 2 mm and the internal diameter approximate the inlet 211 may be around 5 mm. In embodiments where the spiral-shaped conduit 213 has a constant internal diameter between the outlet 212 and the inlet 211, the internal diameter may be around 3 mm.

In some embodiments, the receptacle 220 may include a side wall 221 and a base 222. The inner surface of the base 222 defines a cone shape. Due to the fact that emissions are directly condensed in the receptacle 220 the volumes of collected emissions are relatively low. Advantageously, by providing a cone shaped base 222 it is relatively straightforward to subsequently handle / manipulate the condensed emissions, for example by using a pipette to extract the condensed emissions from the lowermost point of the receptacle 220. Advantageously, this can be done without having to tilt the receptacle 220.

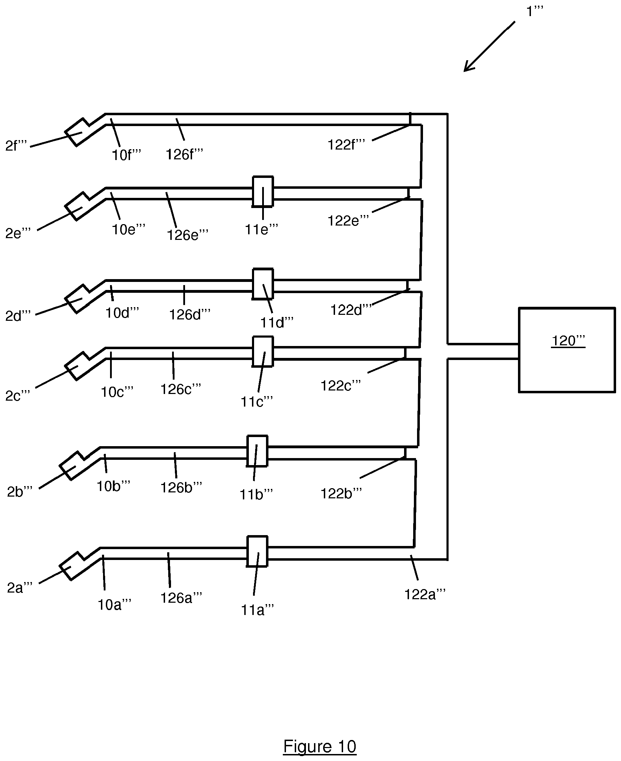

Although in figure 9 the collecting assembly 200 is shown incorporated into the apparatus 1 of figure 1 in place of the collecting assembly 13, the collecting assembly 200 may also be incorporated into other embodiments, for example the apparatus 1' of figure 2 or the apparatus 1" of figure 3 or an apparatus 1‴ of figure 10. Moreover, in some embodiments, collecting assemblies 200 of this type may have the configuration as shown in figure 8, comprising plural receptacles 220 arranged in series. In such cases the conduit 121b may be replaced with or connected to a conduit 121c to allow for the receptacles 220 to be in fluid communication with each other. It can be appreciated that a number of such receptacles can be connected in series.

Figure 10 illustrates an embodiment whereby multiple devices 2a‴-2e‴ can be tested at the same time by utilising multiple fluid lines 126a‴-126e‴. In this embodiment each device 2a‴-2e‴ is in fluid communication with a pump 120‴ and each fluid line 126a‴-126e‴ has a respective valve 122a‴-122e‴ associated therewith. There is also an optional dummy device 2f‴ in fluid communication with the pump 120‴ via a fluid line 126f‴ which has a respective valve 122f‴ associated therewith.

In principal, this apparatus, indicated generally at 1‴, works in the same way as the previously described apparatus. The apparatus 1‴ is shown in a configuration whereby the valve 122a‴ is open and the remaining valves 122b‴-122e‴ are closed. A valve 122f‴ (described later) is also closed. In other words, the valve 122a‴ is in a configuration which permits drawing of emissions by the pump 120‴ from the inlet 10a‴ to the outlet 11a‴ and valves 122b‴-122e" are in configurations which prohibit or substantially prohibit drawing of the emissions by the pump 120‴ from a respective inlet 10b‴-10e‴ to a respective outlet 11b‴-11e‴.

After a predetermined period of time (e.g. 5 seconds) valve 122a‴ closes and valve 122b‴ opens. This has the effect of permitting drawing of emissions by the pump 120‴ from the inlet 10b‴ to the outlet 11b‴ and valves 122a‴, 122c‴-122e‴ are in configurations which prohibit or substantially prohibit drawing of the emissions by the pump 120‴ from a respective inlet 10a‴, 10c‴-10e‴ to a respective outlet 11a‴, 11c‴-11e‴. After a further predetermined period of time (e.g. 5 seconds) valve 122b‴ closes and 122c‴ opens. This can continue until emissions have been drawn from each and every device 2a‴-2e‴. Once emissions have been drawn from each of the devices 2a‴-2e‴ the cycle may be repeated one or more times. Once the cycle has been repeated (such that, say, ten 5 second "puffs" have been drawn from each device 2a‴-2e‴) valves 122a‴-122e‴ may be closed and valve 122f‴ may be opened. This permits fluid to be drawn by the pump 120‴ along the fluid line 126f‴ from inlet 10f‴ (which may or may not have connected thereto a dummy device 2f‴). This configuration (i.e. drawing from fluid line 126f‴) may remain for a predetermined period of time (say 15 minutes) before repeating the "puff cycles" as described above. Thus, in one example we may have a regime as follows:
1) Puff duration: 5 sec (per fluid line 126a‴-126e‴)
2) Interval between each puff: 20 sec
3) Number of cycles per session: 10
4) Interval between sessions: 15 minutes (i.e. when valve 122f‴ is open)
5) Number of sessions: 3 (number of times steps 1 to 4 is repeated)

This, thus, mimics a user who may draw 10 puffs of 5 seconds each time an ENDS is used (i.e. 1 vaping session). Then (say, after 1 hour or so) the user may use the ENDS again with the same parameters. The gap of 15 minutes represents this 1 hour gap of use. For the purposes of testing the 15 minute interval between sessions can be reduced to approximately 5 to 7 minutes as this is sufficient time for the device 2a‴-2e‴ to revert to room temperature.

The apparatus 1‴ can, of course, be generalised to any number of fluid lines 126a‴-126e‴ / devices 2a"'-2e"', but practically this number may be from 1 to 10.

As discussed, a controller 14, 14', 14" may be configured to control the valves 122a‴-122f‴ and / or other components of the apparatus 1‴. The controller 14, 14', 14" may be programmable such that different regimes may be adopted. Any one or more of the puff duration, interval between each puff, number of cycles per session, interval between sessions and number of sessions may deviate from that described herein.

The inventor has noted that when the apparatus 1‴ has adopted step 4 (i.e. when only valve 122f‴ is open) condensed emissions in the collecting assemblies associated with the fluid lines 126a‴-126e‴ adsorb / stick to the internal walls of conduit (particularly the narrowest sections of capillary tubing). This accumulation is unable to drip into the associated receptacle due to it being viscous. This means that when the next session starts the first 1 or 2 puffs do not actually draw any emissions from the device 2a‴-2e‴ but instead are utilised to clear the blockage(s). To counteract this, before starting each session, the controller 14, 14', 14" may first perform a "clear line" sequence, in which each fluid line 126a‴-126e‴ is purged to clear any blockages.

When used in this specification and claims, the terms "comprises" and "comprising" and variations thereof mean that the specified features, steps or integers are included. The terms are not to be interpreted to exclude the presence of other features, steps or components.

## Claims

1. A collecting assembly (200), comprising:
a condensing device (210) including an inlet (211), an outlet (212) and a spiral-shaped conduit (213) extending between the inlet (211) and the outlet (212);
**characterised in that** the collecting assembly (200) further comprises:
a receptacle (220) for collecting emissions from the condensing device (210), the receptacle (220) having a side wall (221) and a base (222), wherein the inner surface of the base (222) defines a cone shape.

2. A collecting assembly (200) according to claim 1, wherein the spiral-shaped conduit (213) comprises a capillary.

3. A collecting assembly (200) according to claim 1 or 2, wherein the spiral-shaped conduit (213) has a narrower internal diameter at the outlet (212) than at the inlet (211).

4. A collecting assembly (200) according to claim 3, wherein the internal diameter reduces gradually along the length of the spiral-shaped conduit (213) from the inlet (211) to the outlet (212).

5. A collecting assembly (200) according to claim 3 or claim 4, wherein the internal diameter of the spiral-shaped conduit (213) is from approximately 2 mm to approximately 10 mm.

6. A collecting assembly (200) according to any one of claims 1 to 5, further comprising a device (230) configured to regulate the temperature of the receptacle (220).

## Patentansprüche

1. Sammelanordnung (200), umfassend:
eine Kondensationsvorrichtung (210), die einen Einlass (211), einen Auslass (212) und eine spiralförmige Leitung (213) mit Erstreckung zwischen dem Einlass (211) und dem Auslass (212) beinhaltet;
**dadurch gekennzeichnet, dass** die Sammelanordnung (200) ferner umfasst:
eine Aufnahme (220) zum Sammeln von Emissionen aus der Kondensationsvorrichtung (210), wobei die Aufnahme (220) eine Seitenwand (221) und eine Basis (222) aufweist, wobei die Innenfläche der Basis (222) eine Kegelform definiert.

2. Sammelanordnung (200) nach Anspruch 1, wobei die spiralförmige Leitung (213) eine Kapillare umfasst.

3. Sammelanordnung (200) nach Anspruch 1 oder 2, wobei die spiralförmige Leitung (213) an dem Auslass (212) einen schmaleren Innendurchmesser als an dem Einlass (211) aufweist.

4. Sammelanordnung (200) nach Anspruch 3, wobei der Innendurchmesser entlang der Länge der spiralförmigen Leitung (213) von dem Einlass (211) zu dem Auslass (212) allmählich abnimmt.

5. Sammelanordnung (200) nach Anspruch 3 oder Anspruch 4, wobei der Innendurchmesser der spiralförmigen Leitung (213) von ungefähr 2 mm bis ungefähr 10 mm beträgt.

6. Sammelanordnung (200) nach einem der Ansprüche 1 bis 5, ferner umfassend eine Vorrichtung (230), die dafür konfiguriert ist, die Temperatur der Aufnahme (220) zu regulieren.

## Revendications

1. Ensemble de collecte (200), comprenant :
un dispositif de condensation (210) comprenant une entrée (211), une sortie (212) et un conduit en forme de spirale (213) s'étendant entre l'entrée (211) et la sortie (212) ;
l'ensemble de collecte (200) étant **caractérisé en ce qu'**il comprend en outre :
un réceptacle (220) pour collecter des émissions en provenance du dispositif de condensation (210), le réceptacle (220) ayant une paroi latérale (221) et une base (222), la surface intérieure de la base (222) définissant une forme de cône.

2. Ensemble de collecte (200) selon la revendication 1, dans lequel le conduit en forme de spirale (213) comprend un capillaire.

3. Ensemble de collecte (200) selon la revendication 1 ou 2, dans lequel le conduit en forme de spirale (213) a un diamètre interne plus étroit au niveau de la sortie (212) qu'au niveau de l'entrée (211).

4. Ensemble de collecte (200) selon la revendication 3, dans lequel le diamètre interne diminue progressivement sur la longueur du conduit en forme de spirale (213) depuis l'entrée (211) jusqu'à la sortie (212).

5. Ensemble de collecte (200) selon la revendication 3 ou 4, dans lequel le diamètre interne du conduit en forme de spirale (213) est d'environ 2 mm à environ 10 mm.

6. Ensemble de collecte (200) selon l'une quelconque des revendications 1 à 5, comprenant en outre un dispositif (230) configuré pour réguler la température du réceptacle (220).
